# EUROPEAN PATENT APPLICATION

(11) **EP 4 548 946 A1**
(43) Date of publication of application: **07.05.2025**
(21) Application number: 23871389.5
(22) Date of filing: 18.07.2023
(51) Int. Cl.: A61M 5/142

(54) **CANNULA PORT, CRADLE DEVICE AND DRUG SOLUTION ADMINISTRATION DEVICE**

(30) Priority: 29.09.2022 JP 2022157066
(71) Applicant: Terumo Kabushiki Kaisha, Tokyo 151-0072 (JP)
(72) Inventor: UCHIYAMA Joji, Ashigarakami-gun, Kanagawa 259-0151 (JP)
(74) Representative: TBK
(86) International application number: PCT/JP2023/026288
(87) International publication number: WO 2024/070145

(57) **Abstract**

A cannula port according to the present disclosure includes a cannula, a cannula housing, and a fixing member. The cannula includes a tube main body portion and an extension portion extending toward the outside in the radial direction from the tube main body portion. The cannula housing defines an accommodation space that accommodates the extension portion of the cannula. The fixing member is located in the accommodation space and sandwiches the extension portion of the cannula with the cannula housing. The fixing member defines an internal flow path in fluid communication with the cannula. The internal flow path includes a straight portion extending linearly in an axial direction of the tube main body portion, and a funnel portion connected to a flow path upstream side of the straight portion and having a flow path diameter gradually increasing toward the flow path upstream side.

## Description

### Technical Field

The present disclosure relates to a cannula port, a cradle device, and a liquid medicine administration device.

### Background Art

In recent years, a treatment method of continuously administering a liquid medicine into the body of a patient by subcutaneous injection, intravenous injection, or the like has been performed. For example, as a treatment method for a diabetic patient, a treatment of continuously injecting a trace amount of insulin into the body of the patient is performed. In this treatment method, in order to administer insulin as a liquid medicine to a patient throughout the day, an insulin pump is used as a portable liquid medicine administration device that can be fixed and carried on the patient's body or clothes. Patent Literature 1 discloses a cannula unit used for this type of insulin pump.

The cannula unit described in Patent Literature 1 includes a cannula and a housing. Patent Literature 1 discloses a configuration in which a cannula is sandwiched between a bore of a first portion of a housing and a bush of a second portion of the housing.

### Citation List

### Patent Literature

Patent Literature 1: WO 2018/033614

### Summary of Invention

### Technical Problem

As in the cannula unit described in Patent Literature 1, the cannula is sandwiched and fixed by the first portion and the second portion of the housing, whereby the cannula is firmly fixed to the housing. However, when the cannula is fixed to the first portion of the housing, if the second portion including the bush as the fixing member described in Patent Literature 1 is adopted, the dead volume in which the liquid medicine is retained in the housing tends to be large. Specifically, in Patent Literature 1, a space in the bush of the second portion becomes the dead volume in which the liquid medicine remains after completion of the liquid medicine administration to the living body. Therefore, it is not preferable from the viewpoint of saving the liquid medicine.

On the other hand, the space in the bush of the second portion of Patent Literature 1 is used not only as a flow path of the liquid medicine but also as a passage hole of an inner needle. The inner needle is inserted into the living body together with the cannula in a state of being inserted into the cannula, and is removed from the cannula after the cannula is indwelled in the living body. When the diameter of the space in the bush of the second portion of Patent Literature 1 is reduced for the purpose of reducing the dead volume described above, it is difficult to insert the inner needle into the space in the bush of the second portion. Even if the inner needle can be inserted into the space in the bush of the second portion, it may be difficult to insert the inner needle into the space in the bush of the second portion due to the sliding resistance between an outer surface of the inner needle and an inner surface of the bush. Furthermore, when removing the inner needle from the cannula after the cannula is indwelled in the living body, the sliding resistance between the outer surface of the inner needle and the inner surface of the bush may increase. As described above, when the diameter of the space in the bush in the second portion of Patent Literature 1 is reduced, the passability of the inner needle becomes a problem.

An object of the present disclosure is to provide a cannula port, a cradle device, and a liquid medicine administration device capable of reducing a dead volume in which a liquid medicine remains and suppressing deterioration of passability of an inner needle.

### Solution to Problem

A cannula port according to a first aspect of the present disclosure is a cannula port including:
(1)
   a cannula that is insertable into a living body from a living body surface;
   a cannula housing that holds the cannula; and
   a fixing member that fixes a position of the cannula with respect to the cannula housing, in which:
      the cannula includes
      a tube main body portion including a distal end capable of puncturing the living body, and
      a funnel-shaped or flange-shaped extension portion extending from a proximal end of the tube main body portion toward an outside in a radial direction of the tube main body portion;
      the cannula housing defines
      an accommodation space that accommodates the extension portion of the cannula, and
      an insertion hole communicating with the accommodation space and through which the tube main body portion of the cannula is inserted;
      the fixing member is located in the accommodation space, and fixes the position of the cannula with respect to the cannula housing by sandwiching the extension portion of the cannula with the cannula housing;
      the fixing member defines an internal flow path in fluid communication with the cannula; and
      the internal flow path includes
      a straight portion linearly extending in an axial direction of the tube main body portion, and
      a funnel portion connected to a flow path upstream side of the straight portion and having a flow path diameter gradually increasing toward the flow path upstream side.

A cannula port according to an embodiment of the present disclosure is
(2)
the cannula port according to (1), further including a septum disposed adjacent to the fixing member so as to cover a removal direction of the fixing member in the accommodation space of the cannula housing when a direction from the distal end toward the proximal end in the axial direction of the tube main body portion is the removal direction.

A cannula port according to an embodiment of the present disclosure is
(3)
the cannula port according to (2), in which:
the cannula housing includes
a housing main body that defines the accommodation space and the insertion hole, and
a connecting portion that is connected to the housing main body and is connected to a device main body including a liquid medicine container capable of storing a liquid medicine;
the connecting portion defines a connection flow path communicating with the accommodation space and extending in a direction orthogonal to the axial direction from the accommodation space; and
the fixing member defines a communication flow path that allows the connection flow path to communicate with the internal flow path.

A cannula port according to an embodiment of the present disclosure is
(4)
the cannula port according to (3), in which
the fixing member includes a groove portion formed in an end surface in the removal direction facing the septum, and
the communication flow path includes a groove space defined by the groove portion.

A cannula port according to an embodiment of the present disclosure is
(5)
the cannula port according to any one of (1) to (4), in which the cannula housing includes, on an inner surface that defines the accommodation space, a locking portion that locks the fixing member.

A cannula port according to an embodiment of the present disclosure is
(6)
the cannula port according to (5), in which the locking portion is
a projection portion protruding from the inner surface defining the accommodation space, or
a recess formed in the inner surface defining the accommodation space.

A cradle device according to a second aspect of the present disclosure is
(7)
a cradle device including
the cannula port according to any one of (1) to (6), and
a base body to which the cannula port is attachable, the base body being attachable to the living body surface.

A liquid medicine administration device according to a third aspect of the present disclosure is
(8)
a liquid medicine administration device including
the cradle device according to (7), and
a device main body including a liquid medicine container capable of storing a liquid medicine and attachable to and detachable from the cradle device.

### Advantageous Effects of Invention

According to the present disclosure, it is possible to provide a cannula port, a cradle device, and a liquid medicine administration device capable of reducing a dead volume in which a liquid medicine remains and suppressing deterioration of passability of an inner needle.

### Brief Description of Drawings

Fig. 1 is a perspective view illustrating a liquid medicine administration device.
Fig. 2 is a perspective view illustrating a state before a device main body and a cradle device of the liquid medicine administration device are attached to each other.
Fig. 3 is a plan view illustrating the cradle device.
Fig. 4 is an explanatory view illustrating a state in the middle of attaching the device main body to the cradle device.
Fig. 5 is a perspective view of a cannula port alone.
Fig. 6 is an exploded perspective view of the cannula port.
Fig. 7 is a cross-sectional view of the cannula port.
Fig. 8A is a diagram illustrating a modification of a fixing member.
Fig. 8B is a diagram illustrating a modification of the fixing member.
Fig. 8C is a diagram illustrating a modification of the fixing member.
Fig. 9 is a diagram illustrating a modification of the fixing member.
Fig. 10A is a diagram illustrating a state before a cannula of a cannula port is inserted into a living body.
Fig. 10B is a diagram illustrating a step of inserting the cannula and an inner needle into the living body from the state illustrated in Fig. 10A.
Fig. 10C is a diagram illustrating a step of removing the inner needle from the cannula from the state illustrated in Fig. 10B.
Fig. 10D is a diagram illustrating a step of attaching the device main body to the base main body from the state illustrated in Fig. 10C.

### Description of Embodiments

Hereinafter, embodiments of a cannula port, a cradle device, and a liquid medicine administration device according to the present disclosure will be described by way of example with reference to the drawings. In the drawings, the same components are denoted by the same reference numerals.

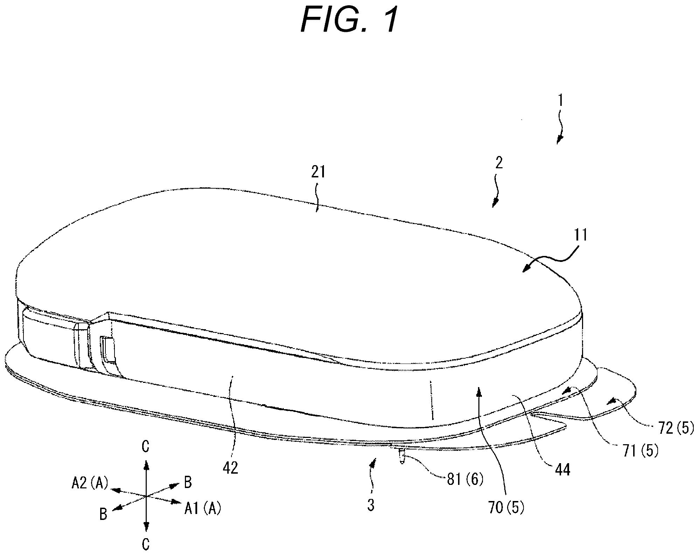

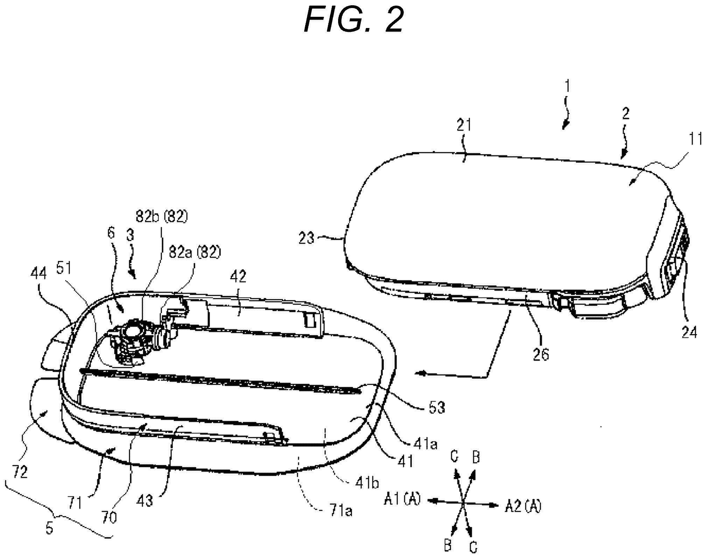

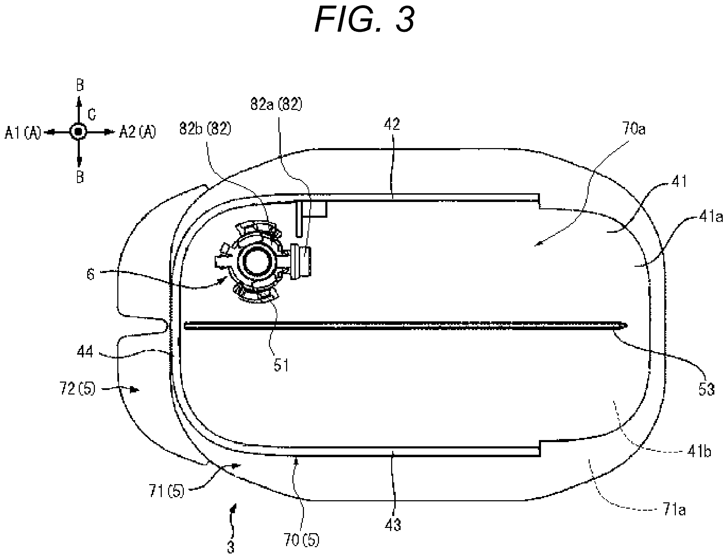

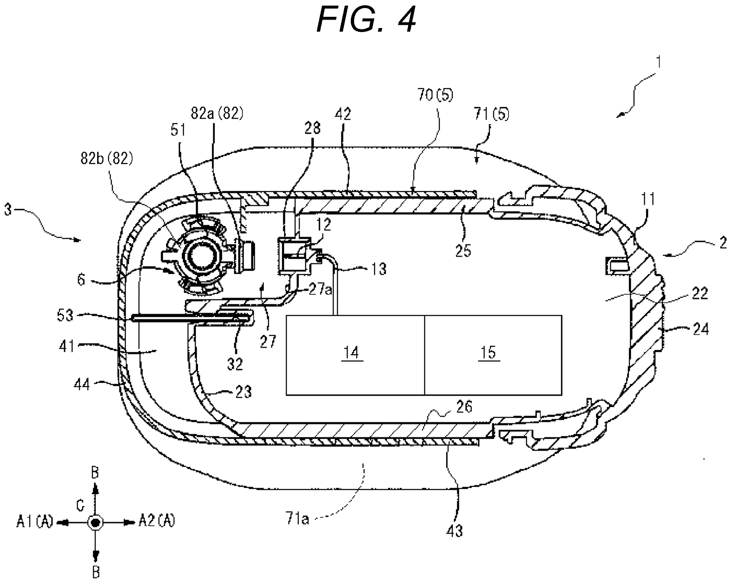

First, a liquid medicine administration device 1 as an embodiment of a liquid medicine administration device according to the present disclosure will be described with reference to Figs. 1 to 4. Fig. 1 is a perspective view illustrating the liquid medicine administration device 1. Fig. 2 is a perspective view illustrating a state before a device main body 2 and a cradle device 3 of the liquid medicine administration device 1 are attached to each other. Fig. 3 is a plan view illustrating the cradle device 3. Fig. 4 is an explanatory view illustrating a state where the device main body 2 is being attached to the cradle device 3.

As illustrated in Fig. 1, the liquid medicine administration device 1 is a portable insulin pump for continuously administering a liquid medicine into a living body such as a patient like a patch-type or tube-type insulin pump and other portable liquid medicine administration devices. The liquid medicine administration device according to the present disclosure is not limited to the insulin pump illustrated in Fig. 1, but in the present embodiment, an insulin pump as an example of the liquid medicine administration device according to the present disclosure will be described by way of example. The liquid medicine administration device 1 includes the device main body 2 and the cradle device 3 configured to be attachable to and detachable from the device main body 2. The cradle device 3 includes a cannula port 6 described later. The cannula port 6 includes a cannula 81 that can be inserted into the living body from the surface of the living body.

### Device main body 2

As illustrated in Figs. 1, 2, and 4, the device main body 2 includes a hollow housing 11. Additionally, as illustrated in Fig. 4, the device main body 2 includes a connecting needle tube 12. Furthermore, as illustrated in Fig. 4, the device main body 2 includes a liquid delivery pipe 13, a liquid medicine container 14, and a drive unit 15 in the housing 11. The liquid medicine container 14 stores a liquid medicine to be administered into a living body. One end portion of the liquid delivery pipe 13 is connected to the liquid medicine container 14. The other end portion of the liquid delivery pipe 13 is connected to the connecting needle tube 12. The drive unit 15 can send the liquid medicine stored in the liquid medicine container 14 into the liquid delivery pipe 13. The liquid medicine sent into the liquid delivery pipe 13 from the liquid medicine container 14 is discharged from the device main body 2 through the connecting needle tube 12. The connecting needle tube 12 is punctured into a connecting portion 82a of the cannula port 6 described later in a state where the device main body 2 is attached to the cradle device 3. As a result, the liquid delivery pipe 13 and the connecting portion 82a are connected via the connecting needle tube 12.

Hereinafter, for convenience of description, a direction in which the device main body 2 is attached to and detached from the cradle device 3 will be referred to as an attaching and detaching direction A. Of the attaching and detaching direction A, a direction in which the device main body 2 is moved with respect to the cradle device 3 at the time of attachment is referred to as an attachment direction A1. Of the attaching and detaching direction A, a direction in which the device main body 2 is moved with respect to the cradle device 3 at the time of detachment is referred to as a detachment direction A2. Additionally, a direction orthogonal to the attaching and detaching direction A and also orthogonal to a direction in which the device main body 2 and the cradle device 3 overlap with each other is referred to as a device width direction B. Moreover, a direction in which the device main body 2 and the cradle device 3 overlap each other, that is, a direction orthogonal to the attaching and detaching direction A and the device width direction B is referred to as a device height direction C.

The housing 11 may be formed in a flat and substantially rectangular parallelepiped shape having curved corners. The housing 11 of the present embodiment includes a top plate portion 21, a bottom plate portion 22, a front plate portion 23, a rear plate portion 24, a first side plate portion 25, and a second side plate portion 26. The top plate portion 21 and the bottom plate portion 22 face each other in the device height direction C. The top plate portion 21 is formed in a substantially rectangular shape with curved corners. Additionally, the central portion of the top plate portion 21 bulges toward the side opposite to the bottom plate portion 22 in the device height direction C.

The front plate portion 23 is substantially vertically continuous to one end portion of the top plate portion 21 in the attaching and detaching direction A, and the rear plate portion 24 is substantially vertically continuous to the other end portion of the top plate portion 21 in the attaching and detaching direction A. The front plate portion 23 and the rear plate portion 24 face each other in the attaching and detaching direction A. The first side plate portion 25 is substantially vertically continuous to one end portion of the top plate portion 21 in the device width direction B, and the second side plate portion 26 is substantially vertically continuous to the other end portion of the top plate portion 21 in the device width direction B.

A sliding groove 32 that accommodates a sliding rail 53, which will be described later, of the cradle device 3, and slides with the sliding rail 53 is formed in the bottom plate portion 22. In addition, the bottom plate portion 22 may be provided with, for example, an attachment detection switch capable of detecting a state in which the device main body 2 is attached to the cradle device 3.

As illustrated in Fig. 4, the housing 11 defines a port accommodating portion 27 capable of accommodating the cannula port 6 described later in a state where the device main body 2 is attached to the cradle device 3. The port accommodating portion 27 is formed in a corner where the bottom plate portion 22, the first side plate portion 25, and the front plate portion 23 in the housing 11 are continuous. The port accommodating portion 27 is a recess recessed by a predetermined length from the bottom plate portion 22 toward the top plate portion 21 along the device height direction C.

A tubular connection port 28 is formed in a side wall 27a defining the detachment direction A2 of the port accommodating portion 27. The connection port 28 protrudes from the side wall 27a in the attachment direction A1. The connecting needle tube 12 is disposed in the cylindrical hole of the connection port 28. The connecting needle tube 12 protrudes from the side wall 27a in the attachment direction A1.

The first side plate portion 25 and the second side plate portion 26 of the housing 11 may be provided with a locking portion that engages with a receiving portion of the cradle device 3 when the device main body 2 is attached to the cradle device 3 and can maintain an attached state of the device main body 2 and the cradle device 3. The locking portion and the receiving portion may include, for example, a locking claw portion and a recess into which the locking claw portion is fitted. The locked state of the device main body 2 and the cradle device 3 by the locking portion and the receiving portion is configured to be releasable by the operation of the operator. Thus, the device main body 2 is configured to be attachable to and detachable from the cradle device 3. However, specific configurations of the locking portion and the receiving portion are not particularly limited. That is, the locking portion and the receiving portion are not limited to the locking claw portion and the recess described above.

### Cradle device 3

As illustrated in Figs. 1 to 4, the cradle device 3 of the present embodiment includes a base body 5 and a cannula port 6.

The base body 5 is configured to be attachable to a surface of a living body. Furthermore, the base body 5 is configured such that the cannula port 6 can be attached thereto. Furthermore, the base body 5 is configured such that the device main body 2 is attachable thereto and detachable therefrom.

More specifically, the base body 5 of the present embodiment includes a base main body 70, an adhesive sheet 71, and release paper 72.

The base main body 70 of the present embodiment includes a substantially flat plate-shaped placement portion 41, a first side wall portion 42, a second side wall portion 43, and a third side wall portion 44. As illustrated in Figs. 2 to 4, the base main body 70 of the present embodiment defines an accommodation space 70a capable of accommodating the device main body 2. The accommodation space 70a is a space surrounded by the placement portion 41, the first side wall portion 42, the second side wall portion 43, and the third side wall portion 44.

The placement portion 41 is formed in a substantially rectangular shape with curved corners. When the device main body 2 is attached to the cradle device 3, the bottom plate portion 22 of the housing 11 of the device main body 2 is placed on the placement portion 41.

An attachment portion 51 and a sliding rail 53 are provided on an upper surface 41a of the placement portion 41 on the accommodation space 70a side. The attachment portion 51 is formed in an end portion of the placement portion 41 in the attachment direction A1 and in one end portion of the placement portion 41 in the device width direction B. The attachment portion 51 is configured such that the cannula port 6 described later can be attached thereto. Furthermore, the attachment portion 51 is provided with an insertion hole through which the cannula 81 of the cannula port 6 described later is inserted.

The sliding rail 53 is formed in an intermediate portion of the upper surface 41a of the placement portion 41 in the device width direction B. The sliding rail 53 extends from one end portion to the other end portion of the upper surface 41a of the placement portion 41 in the attaching and detaching direction A. When the device main body 2 is attached to the cradle device 3, the sliding groove 32 of the device main body 2 is slidably fitted into the sliding rail 53.

The first side wall portion 42 is substantially vertically continuous to one end portion of the placement portion 41 in the device width direction B, and the second side wall portion 43 is substantially vertically continuous to the other end portion of the placement portion 41 in the device width direction B. Then, the third side wall portion 44 is substantially vertically continuous with the end portion of the placement portion 41 in the attachment direction A1. Therefore, three of the four sides of the placement portion 41 are continuous with the first side wall portion 42, the second side wall portion 43, and the third side wall portion 44. Then, no side wall portion is continuous with the end portion of the placement portion 41 in the detachment direction A2. That is, the end portion of the accommodation space 70a in the detachment direction A2 is open. The device main body 2 is inserted into the accommodation space 70a from the open end portion of the accommodation space 70a in the detachment direction A2, and is attached to the base main body 70.

The adhesive sheet 71 is configured to be attachable to the skin of a patient as a living body surface. The adhesive sheet 71 is attached to a lower surface 41b (see Fig. 2) of the placement portion 41 of the base main body 70 on a side opposite to the upper surface 41a. An opening through which the cannula 81 of the cannula port 6 described later passes is formed in the adhesive sheet 71.

Furthermore, the adhesive sheet 71 is formed of a flexible member, and a surface of the adhesive sheet 71 on a side opposite to the placement portion 41 side is an adhesive surface 71a that can be attached to a living body surface. The adhesive surface 71a of the adhesive sheet 71 is covered with the release paper 72 in a state before being adhered to the living body surface. That is, the cradle device 3 peels the release paper 72 from the adhesive sheet 71 to expose the adhesive surface 71a of the adhesive sheet 71, so that the cradle device 3 can be attached to the living body surface.

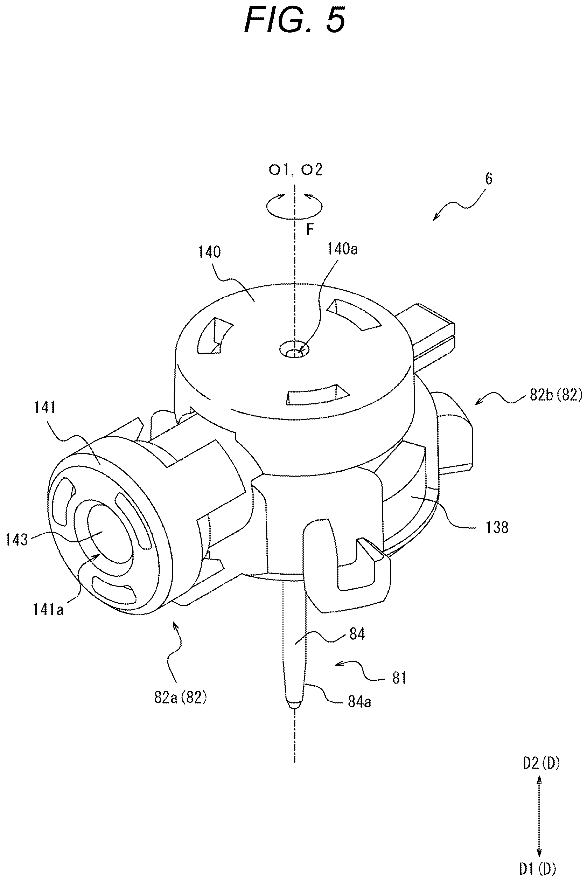

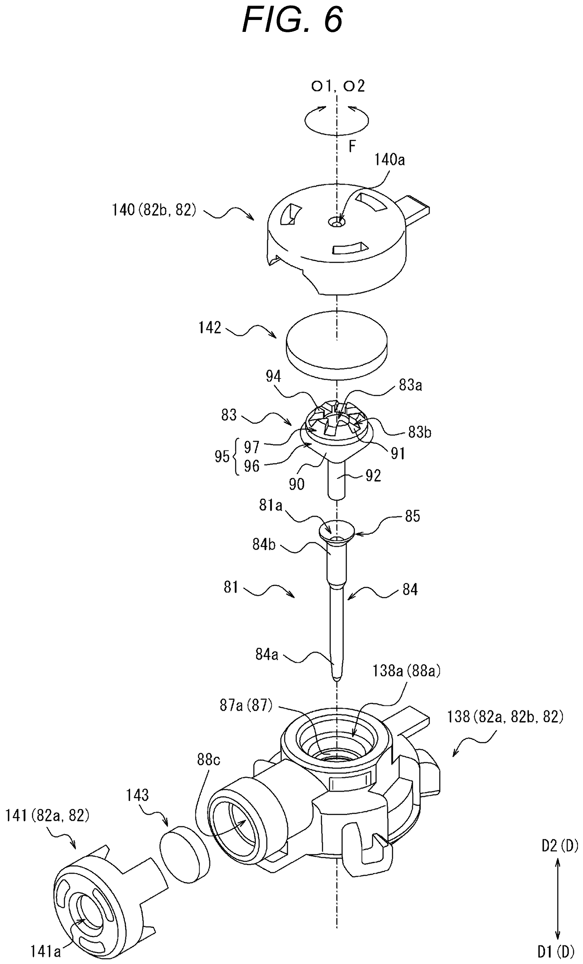

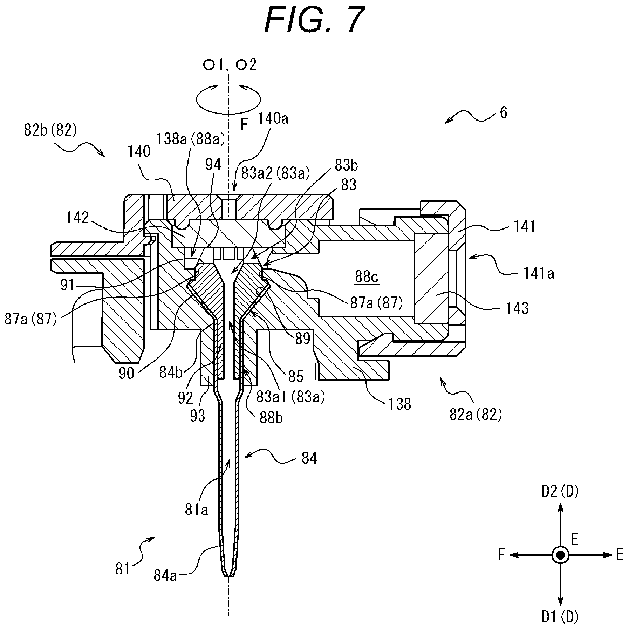

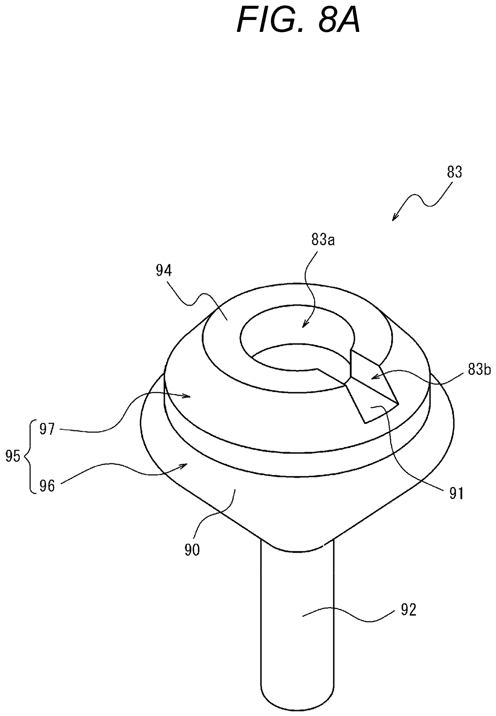

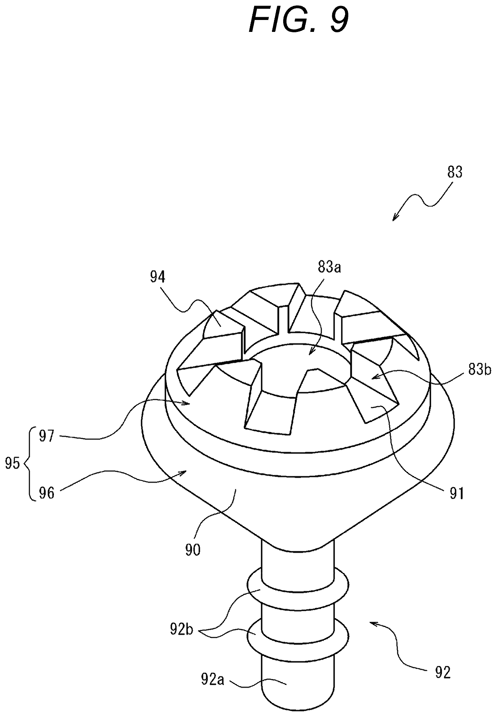

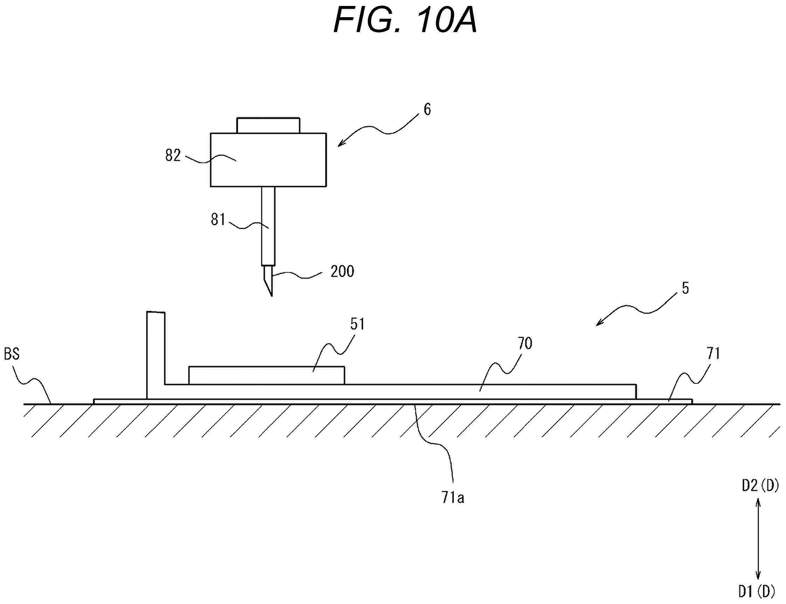

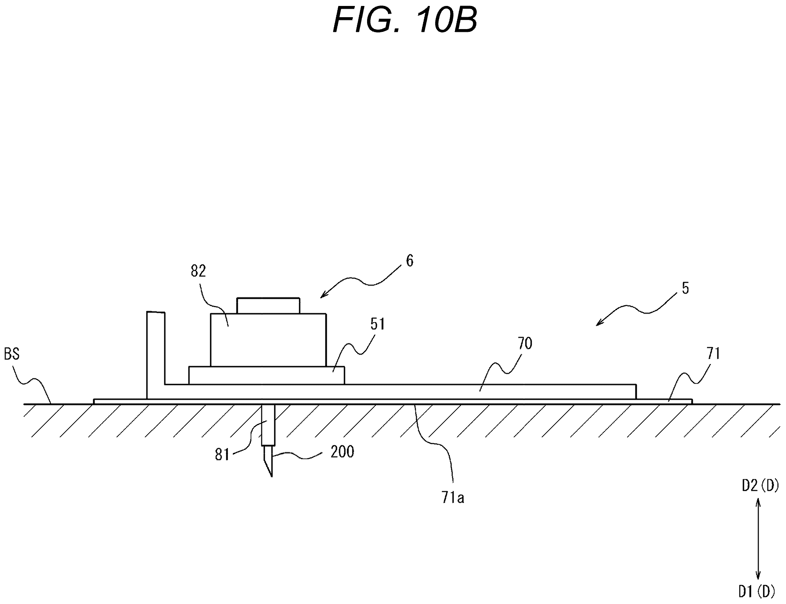

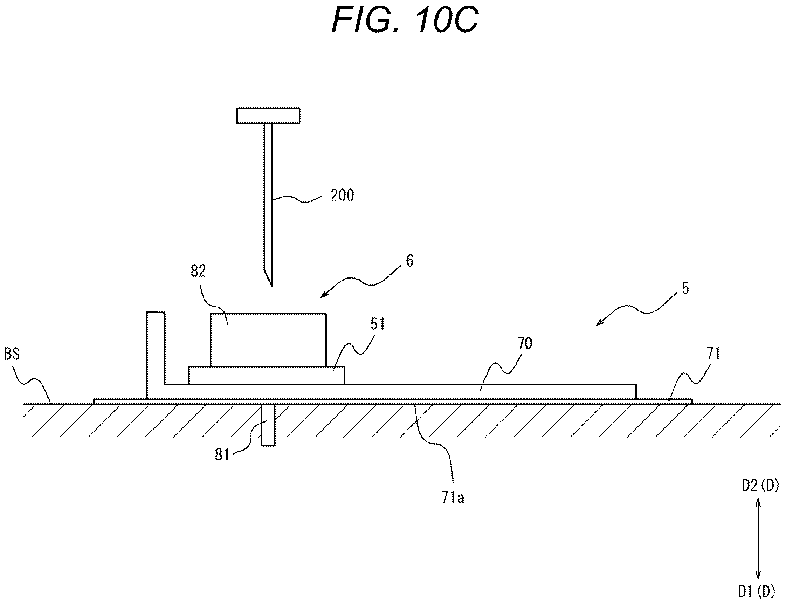

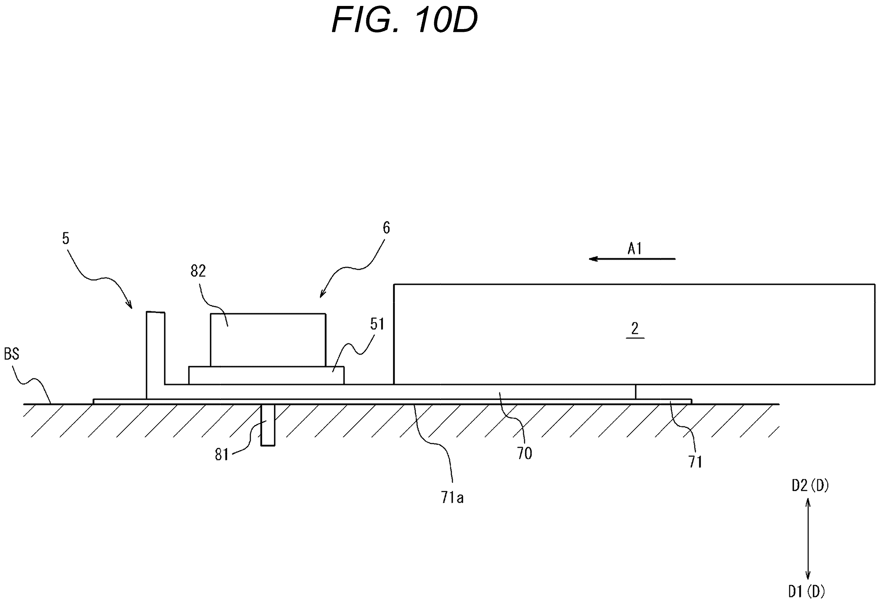

Next, the cannula port 6 will be described with reference to Figs. 2 to 10D. Fig. 5 is a perspective view of the cannula port 6 alone. Fig. 6 is an exploded perspective view of the cannula port 6. Fig. 7 is a cross-sectional view of the cannula port 6 alone. Figs. 8A to 8C are diagrams illustrating modifications of a fixing member 83. Fig. 9 is a diagram illustrating another modification of the fixing member 83. Figs. 10A to 10D are explanatory diagrams illustrating an outline of steps in which the cannula 81 of the cannula port 6 is inserted and indwelled in the living body. Fig. 10A is a diagram illustrating a state before the cannula 81 of the cannula port 6 is inserted into the living body. Fig. 10B is a diagram illustrating a step of inserting the cannula 81 and an inner needle 200 into the living body from the state illustrated in Fig. 10A. Fig. 10C is a diagram illustrating a step of removing the inner needle 200 from the cannula 81 from the state illustrated in Fig. 10B. Fig. 10D is a diagram illustrating a step of attaching the device main body 2 to the base main body 70 from the state illustrated in Fig. 10C. A puncture device is not illustrated in Figs. 10A to 10D.

The cannula port 6 includes the cannula 81 to be inserted into a living body. First, an outline of steps in which the cannula 81 of the cannula port 6 is inserted and indwelled in a living body will be described with reference to Figs. 10A to 10D. As described above, the cannula port 6 is configured to be attachable to the base main body 70. As illustrated in Figs. 10A and 10B, the cannula port 6 is attached to the base main body 70 in a state where the adhesive sheet 71 of the base main body 70 is attached to a living body surface BS. At this time, the cannula 81 of the cannula port 6 penetrates the base main body 70, is inserted into the living body from the living body surface BS, and is indwelled in this state. Attachment of the cannula port 6 to the base main body 70 and insertion of the cannula 81 into the living body can be performed using, for example, a puncture device (not illustrated). The puncture device inserts the cannula 81 into the living body together with the inner needle 200 inserted into the cannula 81. As illustrated in Fig. 10C, the inner needle 200 is removed from the living body after the cannula 81 is inserted and indwelled in the living body. As illustrated in Fig. 10D, the device main body 2 is attached to base main body 70 in this state. At this time, the connecting needle tube 12 (see Fig. 4) of the device main body 2 is connected in fluid communication with the cannula 81 of the cannula port 6. Therefore, the liquid medicine stored in the liquid medicine container 14 (see Fig. 4) of the device main body 2 can be administered into the living body through the liquid delivery pipe 13 (see Fig. 4), the connecting needle tube 12 (see Fig. 4), and the cannula 81.

As illustrated in Figs. 6 and 7, the cannula port 6 includes a cannula housing 82 and the fixing member 83 in addition to the cannula 81 described above. As will be described in detail later, the cannula housing 82 includes a connecting portion 82a. When the device main body 2 is attached to the base main body 70, the connecting needle tube 12 of the device main body 2 punctures the connecting portion 82a of the cannula port 6 in a state of being attached to the base main body 70 in advance. Thereby, as described above, the connecting needle tube 12 of the device main body 2 is connected for fluid communication with the cannula 81 of the cannula port 6. As will be described in detail later, the cannula port 6 may further include a first septum 142 and a second septum 143 as in the present embodiment. Hereinafter, each part of the cannula port 6 will be described.

The cannula 81 is made of resin and has flexibility. As described above, the cannula 81 is configured to be insertable into the living body from the living body surface. Specifically, the cannula 81 is inserted into the living body by the puncture device together with the inner needle 200 (see Figs. 10A to 10C) inserted into the cannula 81. As described above, the inner needle 200 is removed from the living body after the cannula 81 is inserted and indwelled in the living body. In addition, the cannula 81 is removed from the living body together with the cradle device 3 after the liquid medicine administration over a predetermined period, such as several days, is completed.

The cannula 81 includes a tube main body portion 84 and an extension portion 85. The tube main body portion 84 is a portion including a distal end 84a capable of puncturing a living body. The extension portion 85 is a funnel-shaped or flange-shaped (funnel-shaped in the present embodiment) portion extending from a proximal end 84b of the tube main body portion 84 toward the outside in a radial direction E of the tube main body portion 84. Additionally, the extension portion 85 is a portion supported and fixed to the cannula housing 82. Here, the "funnel-shaped extension portion" means a tapered tube portion in which an outer peripheral surface and an inner peripheral surface are inclined with respect to an axial direction D and the outer diameter and the inner diameter are reduced toward an insertion direction D1 when a direction from the proximal end side to the distal end side of the tube main body portion 84 in the axial direction D of the tube main body portion 84 is defined as the insertion direction D1 of the tube main body portion 84. Moreover, the "flange-shaped extension portion" means an annular hollow disk portion protruding outward in the radial direction E in a direction in which a surface orthogonal to the axial direction D extends from the proximal end 84b of the tube main body portion 84.

As illustrated in Fig. 7, the cannula housing 82 holds the cannula 81. As illustrated in Fig. 7, the cannula housing 82 defines an accommodation space 88a that accommodates the extension portion 85 of the cannula 81 and an insertion hole 88b. The insertion hole 88b communicates with the accommodation space 88a. More specifically, the insertion hole 88b allows the accommodation space 88a and the external space outside the cannula housing 82 to communicate with each other. The tube main body portion 84 of the cannula 81 is inserted into the insertion hole 88b in a state where the extension portion 85 is accommodated in the accommodation space 88a. The distal end 84a of the tube main body portion 84 protrudes to the external space outside the cannula housing 82 through the insertion hole 88b.

As illustrated in Fig. 7, the fixing member 83 is located in the accommodation space 88a, and sandwiches the extension portion 85 of the cannula 81 with the cannula housing 82. Thus, the fixing member 83 fixes the position of the cannula 81 with respect to the cannula housing 82. More specifically, the fixing member 83 of the present embodiment includes an inner intubation portion 92 on the puncture direction D1 side and a head portion 95 on a removal direction D2 side. The head portion 95 includes a head main body portion 96 and an end portion 97 continuous with the removal direction D2 side of the head main body portion 96. The head main body portion 96 of the present embodiment includes a tapered outer surface 90 whose diameter expands in the removal direction D2 along a funnel-shaped inner surface reduced diameter portion 89 of the cannula housing 82. The end portion 97 of the present embodiment includes an end surface 94 on which a groove portion 91 is formed. The outer diameter of the end portion 97 is smaller than the maximum outer diameter of the tapered outer surface 90 of the head main body portion 96. As will be described in detail later, in the present embodiment, at least a part of the tapered outer surface 90 of the head main body portion 96 of the head portion 95 and the outer surface of the inner intubation portion 92 are in contact with an inner surface of the cannula 81.

As illustrated in Figs. 6 and 7, the fixing member 83 defines an internal flow path 83a in fluid communication with a cannula flow path 81a of the cannula 81. That is, the liquid medicine stored in the liquid medicine container 14 (see Fig. 4) of the device main body 2 flows into the cannula flow path 81a of the cannula 81 via the internal flow path 83a. As illustrated in Fig. 7, the internal flow path 83a includes a straight portion 83a1 and a funnel portion 83a2. The straight portion 83a1 and the funnel portion 83a2 of the internal flow path 83a are formed by adjusting, in the axial direction D, the thickness of the fixing member 83 orthogonal to the axial direction D. The straight portion 83a1 extends linearly in the axial direction D of the tube main body portion 84 of the cannula 81. An end portion of the straight portion 83a1 in the insertion direction D1 is continuous with the cannula flow path 81a. The end portion of the straight portion 83a1 in the insertion direction D1 does not protrude from a holding block 138. The end portion of the straight portion 83a1 in the insertion direction D1 is located inside the holding block 138. As a result, even if the liquid medicine administration device 1 is pressed toward the skin in a state where the liquid medicine administration device 1 is attached, it is possible to reduce stimulation to the skin around the insertion portion of the cannula 81. Additionally, when a direction from the distal end side toward the proximal end side of the tube main body portion 84 in the axial direction D of the tube main body portion 84 is defined as the removal direction D2 of the tube main body portion 84, an end portion of the straight portion 83a1 in the removal direction D2 is continuous with the funnel portion 83a2. That is, the funnel portion 83a2 is continuous with the flow path upstream side of the straight portion 83a1. The funnel portion 83a2 has a flow path diameter gradually increasing toward the flow path upstream side. Here, the proximal end side of the straight portion 83a1 extends from the inner intubation portion 92 to the head portion 95 in cross-sectional view, and is connected to the funnel portion 83a2 in the head portion 95. Preferably, the funnel portion 83a2 is formed in the end portion 97. Then, the end portion of the funnel portion 83a2 in the removal direction D2 is continuous with the accommodation space 88a.

In this manner, by disposing the fixing member 83 in the accommodation space 88a of the cannula housing 82 and sandwiching the extension portion 85 of the cannula 81 between the cannula housing 82 and the fixing member 83, it is possible to enhance the position fixability of the cannula 81 with respect to the cannula housing 82. Furthermore, by forming the internal flow path 83a in the fixing member 83 disposed in the accommodation space 88a, it is possible to prevent the entire accommodation space 88a from serving as a flow path of the liquid medicine. That is, it is possible to prevent the entire accommodation space 88a from becoming a dead volume in which the liquid medicine remains after the liquid medicine administration to the living body is completed. As a result, the flow path volume through which the liquid medicine can flow in the accommodation space 88a can be reduced, and as a result, the dead volume of the accommodation space 88a can be reduced.

Furthermore, the internal flow path 83a of the fixing member 83 is a flow path of the liquid medicine and is a passage hole of the inner needle 200 described above (see Figs. 10A to 10C). Specifically, the inner needle 200 is inserted into the cannula 81 in advance before the cannula 81 is inserted into the living body. In this state, the inner needle 200 is inserted and passed through the internal flow path 83a of the fixing member 83. Furthermore, as described above, the inner needle 200 is removed from the inside of the cannula 81 after the cannula 81 is inserted and indwelled in the living body. At this time, the inner needle 200 is also removed from the internal flow path 83a of the fixing member 83. Since the internal flow path 83a of the fixing member 83 includes the funnel portion 83a2, it is possible to secure a wide insertion port of the internal flow path 83a for inserting the inner needle 200. This facilitates insertion of the inner needle 200 into the internal flow path 83a. In addition, since the internal flow path 83a includes the straight portion 83a1 and the funnel portion 83a2, it is possible to reduce the sliding resistance between the inner surface defining the internal flow path 83a of the fixing member 83 and the outer surface of the inner needle 200 as compared with a case where the entire internal flow path 83a is configured only by the straight portion 83a1. This facilitates insertion of the inner needle 200 into the internal flow path 83a. In addition, the inner needle 200 can be easily removed from the internal flow path 83a. As described above, since the internal flow path 83a of the fixing member 83 includes the straight portion 83a1 and the funnel portion 83a2, it is possible to suppress deterioration of the passability of the inner needle 200 in the internal flow path 83a. Furthermore, since the internal flow path 83a includes the straight portion 83a1 and the funnel portion 83a2, the dead volume described above can be easily reduced as compared with the case where the entire internal flow path 83a is configured only by the funnel portion 83a2.

As described above, by disposing the fixing member 83 defining the internal flow path 83a including the straight portion 83a1 and the funnel portion 83a2 in the accommodation space 88a, it is possible to reduce a dead volume in which the liquid medicine remains in the accommodation space 88a and to suppress deterioration of the passability of the inner needle 200.

In particular, the diameter of the straight portion 83a1 of the internal flow path 83a of the fixing member 83 is preferably substantially equal to the outer diameter of the inner needle 200 or slightly larger than the outer diameter of the inner needle 200 from the above-described viewpoint of reducing the dead volume and securing the function as the passage hole of the inner needle 200. From the same viewpoint as above, the diameter of the straight portion 83a1 is preferably equal to or less than the maximum inner diameter of the tube main body portion 84 of the cannula 81.

On the other hand, the maximum diameter of the funnel portion 83a2 of the internal flow path 83a of the fixing member 83 is preferably larger than the outer diameter of the inner needle 200 from the viewpoint of suppressing deterioration of the passability of the inner needle 200. In addition, the maximum diameter of the funnel portion 83a2 is preferably larger than the maximum inner diameter of the tube main body portion 84 of the cannula 81 from the viewpoint of suppressing deterioration of the passability of the inner needle 200.

Here, in a state where the fixing member 83 is disposed in the accommodation space 88a, the volume of the space that becomes the flow path of the liquid medicine in the accommodation space 88a is preferably 1/2 or less, more preferably 1/4 or less, and particularly preferably 1/6 or less, compared to the volume of the space that becomes the flow path of the liquid medicine in the accommodation space 88a, in a state where the fixing member 83 is not disposed in the accommodation space 88a. In other words, it is preferable that the size of the fixing member 83 and the volume of the internal flow path 83a of the fixing member 83 satisfy the above-described volume relationship. The volume of the internal flow path 83a may be adjusted by adjusting, in the axial direction D, the thickness of the fixing member 83 orthogonal to the axial direction D. As a result, the dead volume in the accommodation space 88a can be easily reduced.

Hereinafter, further details of the cannula port 6 of the present embodiment will be described.

As illustrated in Figs. 5 to 7, the cannula housing 82 of the present embodiment includes the connecting portion 82a and a housing main body 82b. As illustrated in Fig. 7, the housing main body 82b defines the accommodation space 88a and the insertion hole 88b. The connecting portion 82a is continuous with the housing main body 82b. More specifically, the connecting portion 82a of the present embodiment extends in a direction orthogonal to the axial direction D with respect to the housing main body 82b. As illustrated in Fig. 7, the connecting portion 82a defines a connection flow path 88c that communicates with the accommodation space 88a and extends in a direction orthogonal to the axial direction D from the accommodation space 88a. As described above, the connecting portion 82a is configured to be connectable to the device main body 2.

More specifically, as illustrated in Figs. 5 to 7, the cannula housing 82 of the present embodiment includes the holding block 138, a first cover 140, and a second cover 141.

As illustrated in Fig. 7, the holding block 138 internally defines the accommodation space 88a, the insertion hole 88b, and the connection flow path 88c described above. More specifically, the holding block 138 includes an accommodating recess 138a open in the removal direction D2. The accommodation space 88a is defined by the accommodating recess 138a described above. In addition, a through hole penetrating from a bottom surface of the accommodating recess 138a in the insertion direction D1 is formed in the holding block 138. This through hole is the above-described insertion hole 88b. As will be described in detail later, the bottom surface of the accommodating recess 138a of the present embodiment is configured by the funnel-shaped inner surface reduced diameter portion 89. As illustrated in Fig. 6, the cannula 81 is accommodated in the accommodating recess 138a of the holding block 138 from the open end of the accommodating recess 138a in the removal direction D2. Then, the tube main body portion 84 of the cannula 81 is inserted into the through hole as the insertion hole 88b until the extension portion 85 of the cannula 81 comes into contact with the bottom surface of the accommodating recess 138a. Thus, the cannula 81 can be positioned with respect to the holding block 138. Thereafter, the fixing member 83 is accommodated in the accommodating recess 138a of the holding block 138 from the open end of the accommodating recess 138a in the removal direction D2. When the fixing member 83 is pushed in the insertion direction D1, the extension portion 85 of the cannula 81 is sandwiched between the fixing member 83 and the bottom surface of the accommodating recess 138a. Accordingly, the cannula 81 is fixed to the holding block 138 of the cannula housing 82.

As illustrated in Fig. 7, the removal direction D2 of the accommodating recess 138a of the holding block 138 is covered with the first cover 140 via the first septum 142. That is, as described above, after the cannula 81 is fixed to the holding block 138 using the fixing member 83, the removal direction D2 of the accommodating recess 138a of the holding block 138 is covered with the first cover 140 with the first septum 142 interposed therebetween. Furthermore, as illustrated in Fig. 7, one end portion of the connection flow path 88c defined by the holding block 138 is continuous with the accommodation space 88a, and the other end portion of the connection flow path 88c is open to the outside. The other end portion of the connection flow path 88c is covered with the second cover 141 via the second septum 143. More specifically, the holding block 138 defines a side hole extending in a direction orthogonal to the axial direction D from an inner surface side wall of the accommodating recess 138a. This side hole is the connection flow path 88c described above. The first cover 140 and the second cover 141 are fixed to the holding block 138. In the present embodiment, the holding block 138 and the first cover 140 constitute the housing main body 82b described above. Additionally, in the present embodiment, the holding block 138 and the second cover 141 constitute the connecting portion 82a described above.

As illustrated in Figs. 5 to 7, the above-described first cover 140 is formed with a passage hole 140a through which the above-described inner needle 200 (see Figs. 10A to 10C) can pass. The inner needle 200 is inserted into the accommodation space 88a through the passage hole 140a of the first cover 140 and the first septum 142. The inner needle 200 is removed from the accommodation space 88a through the passage hole 140a of the first cover 140 and the first septum 142. Therefore, after the inner needle 200 is removed, the first septum 142 is closed to secure the liquid tightness of the accommodation space 88a.

As illustrated in Figs. 6 and 7, a central axis O2 of the internal flow path 83a of the fixing member 83 substantially coincides with a central axis O1 of the cannula 81. The central axis O2 of the internal flow path 83a of the fixing member 83 is the central axis of the straight portion 83a1 and the funnel portion 83a2. Moreover, the central axis O1 of the cannula 81 is the central axis of the tube main body portion 84 and the extension portion 85. Furthermore, the passage hole 140a of the first cover 140 is located on the central axis O1 of the cannula 81 and on the central axis O2 of the internal flow path 83a of the fixing member 83. That is, in the present embodiment, the passage hole 140a, the internal flow path 83a, and the cannula flow path 81a are disposed so as to be aligned in a straight line in the axial direction D.

As illustrated in Figs. 5 to 7, the above-described second cover 141 is formed with a passage hole 141a through which the connecting needle tube 12 (see Fig. 4) of the device main body 2 can pass. The connecting needle tube 12 of the device main body 2 is inserted into the connection flow path 88c through the passage hole 141a of the second cover 141 and the second septum 143. As a result, the liquid medicine accommodated in the liquid medicine container 14 (see Fig. 4) of the device main body 2 can be administered into the living body through the liquid delivery pipe 13 (see Fig. 4), the connecting needle tube 12, the connection flow path 88c, the internal flow path 83a of the fixing member 83 located in the accommodation space 88a, and the cannula 81. Additionally, the connecting needle tube 12 of the device main body 2 is removed from the connection flow path 88c through the passage hole 141a of the second cover 141 and the second septum 143. Therefore, after the connecting needle tube 12 is removed, the second septum 143 is closed to secure the liquid tightness of the accommodation space 88a. In the present embodiment, the device main body 2 and the cannula 81 are fluidly connected by the second septum 143 such as silicone rubber and the connecting needle tube 12, and simple and reliable fluid connection is possible. However, the fluid connection method between the device main body 2 and the cannula 81 is not limited thereto, and various needleless connection methods (ball valve type) are also applicable. Therefore, the internal volume of the connection flow path 88c depends on these connection methods.

As described above, in the cannula housing 82 of the present embodiment, the passage hole 140a of the inner needle 200 (Figs. 10A to 10C) used to insert and indwell the cannula 81 into the living body and the passage hole 141a used to connect the connecting needle tube 12 of the device main body 2 are separately provided, but the present invention is not limited to this configuration. That is, the passage hole of the inner needle 200 used to insert and indwell the cannula 81 into the living body may also be used as the passage hole used to connect the connecting needle tube 12 of the device main body 2. In this case, the second septum 143, the second cover 141, and the connection flow path 88c are not provided. In addition, the connecting needle tube 12 is connected via the first septum 142.

As illustrated in Fig. 7, an inner surface of the housing main body 82b of the cannula housing 82, which defines the accommodation space 88a, includes a funnel-shaped inner surface reduced diameter portion 89 whose inner diameter is reduced in the insertion direction D1. The funnel-shaped inner surface reduced diameter portion 89 of the present embodiment is formed on the bottom surface of the accommodating recess 138a of the holding block 138. The inner surface reduced diameter portion 89 can support the funnel-shaped extension portion 85 of the cannula 81 in the removal direction D2 by coming into contact with an outer peripheral surface of the extension portion 85. In this manner, the funnel-shaped extension portion 85 of the cannula 81 is supported by the funnel-shaped inner surface reduced diameter portion 89 of the cannula housing 82, whereby the positioning of the funnel-shaped extension portion 85 in the accommodation space 88a is facilitated. More specifically, the funnel-shaped extension portion 85 can be easily positioned in the accommodating recess 138a of the holding block 138.

Moreover, the difference between the inclination angle of an outer peripheral surface of the funnel-shaped extension portion 85 with respect to the axial direction D and the inclination angle of the inner surface reduced diameter portion 89 with respect to the axial direction D is preferably 1 degree or less. The "inclination angle with respect to the axial direction D" mentioned here refers to an acute inclination angle with respect to the axial direction D. In this way, the contact area of the outer peripheral surface of the funnel-shaped extension portion 85 and the inner surface reduced diameter portion 89 in the axial direction D can be increased. Therefore, the funnel-shaped extension portion 85 can be more easily positioned in the accommodation space 88a.

As illustrated in Figs. 6 and 7, the fixing member 83 of the present embodiment includes the funnel-shaped tapered outer surface 90 whose diameter decreases in the insertion direction D1. Then, as illustrated in Fig. 7, the funnel-shaped extension portion 85 of the cannula 81 is sandwiched between the tapered outer surface 90 of the fixing member 83 and the inner surface reduced diameter portion 89 of the cannula housing 82. The tapered outer surface 90 of the fixing member 83 of the present embodiment has a shape substantially along the inner surface shape of the accommodating recess 138a described later. More specifically, the fixing member 83 of the present embodiment is disposed in a state of being inserted into the funnel-shaped extension portion 85 of the cannula 81 in the accommodation space 88a. In this state, the funnel-shaped extension portion 85 of the cannula 81 is sandwiched between the tapered outer surface 90 of the fixing member 83 and the inner surface reduced diameter portion 89 of the cannula housing 82.

As illustrated in Fig. 7, in the present embodiment, only a part of the fixing member 83 is inserted into the funnel-shaped extension portion 85 of the cannula 81 in the accommodation space 88a. In other words, the fixing member 83 of the present embodiment includes a part not inserted into the extension portion 85 in the accommodation space 88a. In the present embodiment, the part of the fixing member 83 that is not inserted into the extension portion 85 in the accommodation space 88a is pressed in the insertion direction D1 by a projection portion 87a of the cannula housing 82. The projection portion 87a protrudes from an inner surface of the cannula housing 82 that defines the accommodation space 88a. More specifically, in the present embodiment, the projection portion 87a is formed on an inner surface side wall of the accommodating recess 138a of the holding block 138. The fixing member 83 slides on the projection portion 87a and is pushed into the accommodating recess 138a in the insertion direction D1 so as to go over the projection portion 87a. As a result, a state in which the fixing member 83 is pressed in the insertion direction D1 by the projection portion 87a of the cannula housing 82 can be achieved. In other words, a state in which the fixing member 83 does not move in the removal direction D2 can be achieved by abutting on the projection portion 87a of the cannula housing 82. That is, in the present embodiment, the above-described projection portion 87a constitutes a locking portion 87 of the cannula housing 82 that locks the fixing member 83.

The projection portion 87a as the locking portion 87 of the cannula housing 82 of the present embodiment is an annular projection extending over the entire region in a circumferential direction F of the tube main body portion 84, but is not limited to this configuration. The projection portion 87a may be constituted by, for example, a plurality of projections intermittently disposed in the circumferential direction F of the tube main body portion 84. Moreover, the locking portion 87 of the cannula housing 82 is not limited to the projection portion 87a described above. The locking portion 87 of the cannula housing 82 may be constituted by, for example, a recess formed in an inner surface of the cannula housing 82, which defines the accommodation space 88a. Examples of the recess formed in the inner surface of the cannula housing 82 that defines the accommodation space 88a include an annular groove extending over the entire region in the circumferential direction F of the tube main body portion 84. That is, the fixing member 83 is configured such that a part thereof enters an annular groove formed in the inner surface of the cannula housing 82 that defines the accommodation space 88a. As a result, a state in which the fixing member 83 is pressed in the insertion direction D1 and a state in which the fixing member 83 does not move in the removal direction D2 may be achieved.

In addition, as illustrated in Fig. 7, the removal direction D2 of the fixing member 83 is covered with the first septum 142. More specifically, the first septum 142 is disposed adjacent to the fixing member 83 so as to cover the removal direction D2 of the fixing member 83 in the accommodation space 88a of the cannula housing 82. In particular, it is preferable that an edge portion around an opening end in the removal direction D2 of the internal flow path 83a as the end surface 94 in the removal direction D2 of the fixing member 83 is in contact with or close to the first septum 142. "Close" means, for example, being disposed with a gap of 1 mm or less. As described above, by disposing the end surface 94 of the fixing member 83 in the removal direction D2 adjacent to the first septum 142, when the inner needle 200 (see Figs. 10A to 10C) is inserted into the internal flow path 83a through the first septum 142, the first septum 142 comes into contact with the end surface 94 of the fixing member 83 in the removal direction D2 and is supported. As a result, it is possible to prevent the first septum 142 from being deformed to be warped in the insertion direction D1. Therefore, when the inner needle 200 (see Figs. 10A to 10C) is inserted into the internal flow path 83a through the first septum 142, it is possible to suppress the first septum 142 from escaping in the insertion direction D1 and suppress the difficulty of the inner needle 200 puncturing the first septum 142.

In addition, unlike the present embodiment, it is assumed that a configuration in which the passage hole 140a of the first cover 140 and the first septum 142 described above are used for connecting the connecting needle tube 12 of the device main body 2 is adopted. Even in such a case, when the connecting needle tube 12 is inserted into the internal flow path 83a through the first septum 142, even if the first septum 142 escapes in the insertion direction D1 and warps, the end surface 94 on the proximal end side of the fixing member 83 supports the first septum 142 from below from the internal flow path 83a side, so that it is possible to suppress the difficulty of the connecting needle tube 12 puncturing the first septum 142. In addition, even when insertion and removal of the connecting needle tube 12 are repeated, the puncture position of the connecting needle tube 12 in the first septum 142 can be kept substantially constant. That is, even when insertion and removal of the connecting needle tube 12 with respect to the first septum 142 are repeated, such as when the device main body 2 is attached to and detached from the cradle device 3, it is possible to suppress formation of a plurality of puncture marks in the first septum 142. Therefore, it is possible to suppress a decrease in liquid tightness of the accommodation space 88a due to the first septum 142.

As described above, in the present embodiment, the end surface 94 of the fixing member 83 in the removal direction D2 is disposed in contact with or close to the first septum 142. With this configuration, the liquid medicine supplied from the device main body 2 through the connection flow path 88c described above is less likely to enter the internal flow path 83a of the fixing member 83. Therefore, as illustrated in Fig. 6, the fixing member 83 preferably defines a communication flow path 83b that allows the connection flow path 88c to communicate with the internal flow path 83a. By providing such a communication flow path 83b, even when the end surface 94 of the fixing member 83 in the removal direction D2 is disposed in contact with or close to the first septum 142, the liquid medicine supplied from the device main body 2 to the connection flow path 88c can be easily caused to flow into the internal flow path 83a through the communication flow path 83b.

Specifically, the end portion 97 of the fixing member 83 of the present embodiment includes the groove portion 91 formed in the end surface 94 in the removal direction D2 facing the first septum 142. More specifically, a plurality of (six in the present embodiment) groove portions 91 are formed at intervals in the circumferential direction F at an edge portion around the opening end in the removal direction D2 of the internal flow path 83a as the end surface 94 in the removal direction D2 of the fixing member 83 of the present embodiment. The groove portion 91 communicates with the connection flow path 88c outside in the radial direction E. The groove portion 91 communicates with the internal flow path 83a inside in the radial direction E. Therefore, the liquid medicine supplied from the device main body 2 to the connection flow path 88c can flow into the internal flow path 83a through the groove space defined by the groove portion 91. That is, in the present embodiment, the groove space defined by the groove portion 91 constitutes the communication flow path 83b described above.

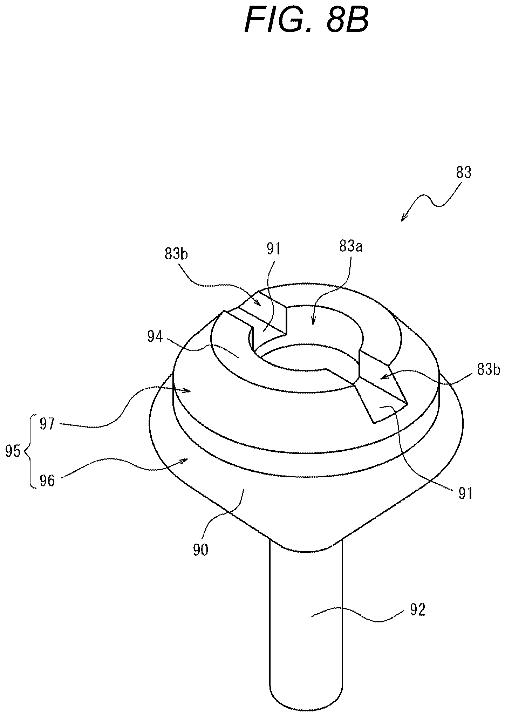

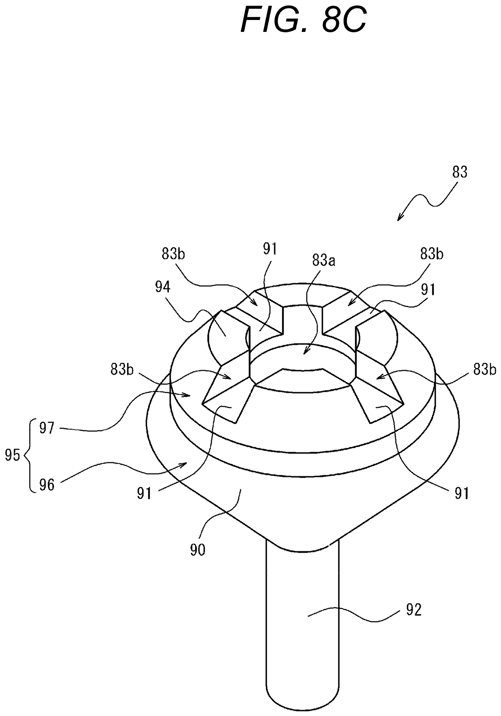

In the present embodiment, the fixing member 83 includes the six groove portions 91, but the present invention is not limited to this configuration. The number of the groove portions 91 of the fixing member 83 may be only one as illustrated in Fig. 8A, or may be less or more than six (two in Fig. 8B and four in Fig. 8C) as illustrated in Figs. 8B and 8C.

In addition, as illustrated in Figs. 6 and 7, the fixing member 83 of the present embodiment includes the inner intubation portion 92 to be inserted into the tube main body portion 84 of the cannula 81. The inner intubation portion 92 internally defines a part of the straight portion 83a1 of the internal flow path 83a described above. The inner intubation portion 92 may be disposed such that its outer surface is in contact with the inner surface of the tube main body portion 84 of the cannula 81. Since the fixing member 83 includes the inner intubation portion 92, bending of the tube main body portion 84 of the cannula 81 can be suppressed. The fixing member 83 may be made of metal or resin. Note, however, that the fixing member 83 is preferably made of resin. The resin inner intubation portion 92 easily follows bending deformation of the tube main body portion 84 of the resin cannula 81 as compared with a metal inner intubation portion. That is, even if the tube main body portion 84 of the resin cannula 81 is bent and deformed by, for example, body motion or the like, the resin inner intubation portion 92 is easily deformed following the deformation of the tube main body portion 84. Therefore, even if the tube main body portion 84 of the resin cannula 81 is bent and deformed, it is possible to suppress generation of a gap between the inner surface of the tube main body portion 84 of the cannula 81 and the outer surface of the inner intubation portion 92 of the fixing member 83. As a result, it is possible to prevent the liquid medicine from entering and being retained between the inner surface of the tube main body portion 84 of the cannula 81 and the outer surface of the inner intubation portion 92 of the fixing member 83. Furthermore, since the fixing member 83 is made of resin, for example, the fixing member 83 does not become an obstacle in X-ray inspection or the like.

Moreover, the tube main body portion 84 of the cannula 81 may be sandwiched between the inner intubation portion 92 of the fixing member 83 and the inner surface of the cannula housing 82 that defines the insertion hole 88b. In other words, the inner intubation portion 92 of the fixing member 83 of the present embodiment may be a caulking pin that clamps the tube main body portion 84 with the inner surface of the cannula housing 82 that defines the insertion hole 88b. As described above, the cannula 81 may be configured such that, not only the extension portion 85 described above, but also the tube main body portion 84 is sandwiched between the cannula housing 82 and the fixing member 83. In this manner, the position fixability of the cannula 81 with respect to the cannula housing 82 can be further improved.

More specifically, the cannula housing 82 of the present embodiment includes a cylindrical portion 93 protruding in the insertion direction D1. Then, the insertion hole 88b is defined inside the cylindrical portion 93. Therefore, the tube main body portion 84 may be sandwiched between an outer surface of the inner intubation portion 92 of the fixing member 83 and an inner surface of the cylindrical portion 93 of the cannula housing 82.

Additionally, as illustrated in Fig. 7, in the tube main body portion 84 of the present embodiment, the inner diameter and the outer diameter of the proximal side portion are larger than the inner diameter and the outer diameter of the distal side portion. The proximal side portion of the tube main body portion 84 is a part where the inner intubation portion 92 is inserted and the periphery thereof is covered with the cylindrical portion 93. The distal side portion of the tube main body portion 84 is a part that is located in the insertion direction D1 from the proximal side portion and can be inserted into the living body. Note, however, that the tube main body portion 84 is not limited to the configuration of the present embodiment. The inner diameter and the outer diameter of the proximal side portion of the tube main body portion 84 do not need to be larger than the inner diameter and the outer diameter of the distal side portion.

Furthermore, as illustrated in Fig. 9, the inner intubation portion 92 may include a tube portion 92a and a protrusion portion 92b protruding in the radial direction from the tube portion 92a. Since the inner intubation portion 92 includes the protrusion portion 92b, the tube main body portion 84 can be more firmly clamped. The protrusion portion 92b illustrated in Fig. 9 is an annular protrusion extending over the entire circumferential direction of the tube portion 92a, but is not limited to this configuration. The protrusion portion 92b may be, for example, a spiral protrusion extending over the entire circumferential direction of the tube portion 92a. Although a plurality of (two in Fig. 9) protrusion portions 92b illustrated in Fig. 9 are disposed at different positions in the axial direction of the tube portion 92a, the number of protrusion portions 92b is not particularly limited. Only one protrusion portion 92b may be provided, or three or more protrusions may be disposed in different positions in the axial direction of the tube portion 92a.

As described above, the fixing member 83 of the present embodiment includes the inner intubation portion 92, but is not limited to this configuration. For example, the fixing member 83 may be configured to be accommodated only in the accommodation space 88a without including the inner intubation portion 92.

The cannula port, the cradle device, and the liquid medicine administration device according to the present disclosure are not limited to the specific configurations described in the above-described embodiments, and various modifications and variations can be made without departing from the scope of the claims.

### Industrial Applicability

The present disclosure relates to a cannula port, a cradle device, and a liquid medicine administration device.

### Reference Signs List

- 1: Liquid medicine administration device
- 2: Device main body
- 3: Cradle device
- 5: Base body
- 6: Cannula port
- 11: Housing
- 12: Connecting needle tube
- 13: Liquid delivery pipe
- 14: Liquid medicine container
- 15: Drive unit
- 21: Top plate portion
- 22: Bottom plate portion
- 23: Front plate portion
- 24: Rear plate portion
- 25: First side plate portion
- 26: Second side plate portion
- 27: Port accommodating portion
- 27a: Side wall
- 28: Connection port
- 32: Sliding groove
- 41: Placement portion
- 41a: Upper surface of placement portion
- 41b: Lower surface of placement portion
- 42: First side wall portion
- 43: Second side wall portion
- 44: Third side wall portion
- 51: Attachment portion
- 53: Sliding rail
- 70: Base main body
- 70a: Accommodation space
- 71: Adhesive sheet
- 71a: Adhesive surface
- 72: Release paper
- 81: Cannula
- 81a: Cannula flow path
- 82: Cannula housing
- 82a: Connecting portion
- 82b: Housing main body
- 83: Fixing member
- 83a: Internal flow path
- 83a1: Straight portion
- 83a2: Funnel portion
- 83b: Communication flow path
- 84: Tube main body portion
- 84a: Distal end
- 84b: Proximal end
- 85: Extension portion
- 87: Locking portion
- 87a: Projection portion
- 88a: Accommodation space
- 88b: Insertion hole
- 88c: Connection flow path
- 89: Inner surface reduced diameter portion
- 90: Tapered outer surface
- 91: Groove portion
- 92: Inner intubation portion
- 92a: Tube portion
- 92b: Protrusion portion
- 93: Cylindrical portion
- 94: End surface of fixing member
- 95: Head portion
- 96: Head main body portion
- 97: End portion
- 138: Holding block
- 138a: Accommodating recess
- 140: First cover
- 140a: Passage hole
- 141: Second cover
- 141a: Passage hole
- 142: First septum
- 143: Second septum
- 200: Inner needle
- A: Attaching and detaching direction
- A1: Attachment direction
- A2: Detachment direction
- B: Device width direction
- BS: Living body surface
- C: Device height direction
- D: Axial direction of tube main body portion
- D1: Insertion direction of tube main body portion
- D2: Removal direction of tube main body portion
- E: Radial direction of tube main body portion
- F: Circumferential direction of tube main body portion
- O1: Central axis of cannula
- O2: Central axis of internal flow path of fixing member

## Claims

1. A cannula port comprising:
a cannula that is insertable into a living body from a living body surface;
a cannula housing that holds the cannula; and
a fixing member that fixes a position of the cannula with respect to the cannula housing, wherein:
the cannula includes
a tube main body portion including a distal end capable of puncturing the living body, and
a funnel-shaped or flange-shaped extension portion extending from a proximal end of the tube main body portion toward an outside in a radial direction of the tube main body portion;
the cannula housing defines
an accommodation space that accommodates the extension portion of the cannula, and
an insertion hole communicating with the accommodation space and through which the tube main body portion of the cannula is inserted;
the fixing member is located in the accommodation space, and fixes the position of the cannula with respect to the cannula housing by sandwiching the extension portion of the cannula with the cannula housing;
the fixing member defines an internal flow path in fluid communication with the cannula; and
the internal flow path includes
a straight portion linearly extending in an axial direction of the tube main body portion, and
a funnel portion connected to a flow path upstream side of the straight portion and having a flow path diameter gradually increasing toward the flow path upstream side.

2. The cannula port according to claim 1, further comprising a septum disposed adjacent to the fixing member so as to cover a removal direction of the fixing member in the accommodation space of the cannula housing when a direction from the distal end toward the proximal end in the axial direction of the tube main body portion is the removal direction.

3. The cannula port according to claim 2, wherein:
the cannula housing includes
a housing main body that defines the accommodation space and the insertion hole, and
a connecting portion that is connected to the housing main body and is connected to a device main body including a liquid medicine container capable of storing a liquid medicine;
the connecting portion defines a connection flow path communicating with the accommodation space and extending in a direction orthogonal to the axial direction from the accommodation space; and
the fixing member defines a communication flow path that allows the connection flow path to communicate with the internal flow path.

4. The cannula port according to claim **3,** wherein
the fixing member includes a groove portion formed in an end surface in the removal direction facing the septum, and
the communication flow path includes a groove space defined by the groove portion.

5. The cannula port according to any one of claims 1 to **4,** wherein the cannula housing includes, on an inner surface that defines the accommodation space, a locking portion that locks the fixing member.

6. The cannula port according to claim **5,** wherein the locking portion is
a projection portion protruding from the inner surface defining the accommodation space, or
a recess formed in the inner surface defining the accommodation space.

7. A cradle device comprising
the cannula port according to any one of claims 1 to 4, and
a base body to which the cannula port is attachable, the base body being attachable to the living body surface.

8. A liquid medicine administration device comprising
the cradle device according to claim 7, and
a device main body including a liquid medicine container capable of storing a liquid medicine and attachable to and detachable from the cradle device.
